# DEMANDE DE BREVET EUROPEEN

(11) **EP 3 275 449 A2**
(43) Date de publication de la demande: **31.01.2018**
(21) Numéro de dépôt: 17183807.1
(22) Date de dépôt: 28.07.2017
(51) Int. Cl.: A61K 35/32, B02C 18/10, B02C 18/30, B02C 18/36, A61K 35/34, A61K 35/35, A61K 35/36, A61P 21/00, A61P 19/00

(54) **SYSTÈME ET PROCÉDÉ DE RÉGÉNÉRATION MUSCLAIRE, TENDINEUSE, CARTILAGINEUSE ET OSSEUSE AU MOYEN DE MICROGREFFES AUTOLOGUES DE CELLULES SOUCHES, PROGÉNITRICES, ET DE CELLULES DU SYSTÈME VASCULO STROMAL, ET UTILISATION DE CEUX-CI**

(30) Priorité: 29.07.2016 ES 201600624
(71) Demandeur: Casanova Rosell, Jose Miguel, 08190 Sant Cugat del Valles Barcelona (ES)
(72) Inventeur: Casanova Rosell, Jose Miguel, 08190 Sant Cugat del Valles Barcelona (ES)
(74) Mandataire: Jeannet, Olivier

(57) **Abrégé**

Le système et le procédé pour la régénération de ces tissus sont basés sur la désagrégation mécanique de tissus autologues tel que derme, muscle, périchondre et cartilage de cellules souches et d'autres composants cellulaires du système vasculo stromal (SVE), obtenus par traitement mécanique de l'échantillon, et comprenant le fait, tout d'abord, d'une désagrégation du tissu, puis le filtrage des cellules souches, suivi de la désinfection de la zone à traiter et de l'application de la solution obtenue le plus près de la zone lésée, ou par injection intra articulaire si la lésion est située à l'intérieur d'une zone d'articulation. Est également concernée l'utilisation des micro-greffes ainsi obtenues pour la thérapie régénérative traumatologique, la réadaptation et la médecine du sport.

## Description

### OBJET DE L'INVENTION

La présente invention concerne un système et un procédé pour la régénération de muscles, de tendons, de cartilage et d'os au moyen de microgreffes autologues de cellules souches, progénitrices, et de cellules du système de vasculo stromal, le système comprenant une machine fonctionnant à une vitesse constante d'environ quatre-vingts tours par minute, qui fonctionne conjointement avec un kit, lequel est un dispositif médical jetable avec certification CE, comprenant une grille stationnaire en acier inoxydable ayant environ cent orifices hexagonaux et, autour de chaque orifice, six microlames, qui effectue la coupe et le filtrage des tissus durs et mous. Un autre objet de l'invention est l'utilisation de ces cellules souches comme microgreffes autologues pour la régénération des tissus concernés.

Dans tous les tissus, tels que la peau ou les follicules pileux, les muscles, le périchondre du cartilage facial, la graisse, etc., il y a une grande quantité de cellules souches, qui ont une fonction réparatrice-régénérative, et c'est pour cette raison qu'il est logique de les appliquer dans des tissus nécessitant une régénération, pour tout type de lésion.

Les avantages de la présente invention par rapport à ce qui est connu antérieurement sont les suivants :
- le procédé est très efficace à l'égard des signes cliniques tels que la douleur, la raideur et l'inflammation, ralentissant ceux-ci et faisant que les tissus traités se revitalisent et se régénèrent dans un stade initial, non-cicatricielle.
- Une session est suffisante si le diagnostic et la technique sont mis en oeuvre correctement, et étant à répéter en cas de réapparition des signes cliniques de la maladie.
- Le procédé est mis en oeuvre rapidement, le patient pouvant reprendre ses activités après seulement 30 minutes.
- Il peut être mis en oeuvre en consultation médicale, sans chirurgie ou chambre stérile, bien que dans un cadre stérile.
- La manipulation du tissu est brève et simple, ne dépassant pas 90 secondes.
- Le procédé n'a pas d'effets secondaires ni de séquelles.
- Il est beaucoup plus économique que tout autre procédé connu.

L'application industrielle de la présente invention se fait dans le domaine de la médecine régénérative traumatologique, réhabilisatrice et de la médecine du sport et en particulier dans la médecine régénérative par microgreffes autologues de cellules souches.

### ARRIÈRE-PLAN DE L'INVENTION

Bien que l'on n'ait relevé aucun procédé identique à celui décrit, il est exposé ci-après différents documents relevés qui reflètent l'art antérieur lié à celui-ci.

Ainsi, le document ES2347078T3 concerne un matériau implantable comprenant une matrice biocompatible et des cellules endothéliales ancrées ou incluses, des cellules de type endothéliales ou analogues destinées à être utilisées pour réduire une réponse immunitaire ou une réaction inflammatoire dans un procédé comprenant l'étape consistant à fournir ledit matériau implantable à un receveur en une quantité suffisante pour réduire la réponse immunitaire ou la réaction inflammatoire chez ledit receveur, la matrice ayant une configuration tridimensionnelle telle que les cellules ancrées et / ou incluses peuvent créer et occuper un habitat multidimensionnel. Dans ce cas, le matériau implantable procède d'une matrice biocompatible, contrairement à l'invention proposée, qui se rapporte à des microgreffes autologues de cellules parentes et régénératrices.

Le document ES2364957T3 propose l'utilisation d'une population concentrée de cellules régénératives obtenues depuis un tissu adipeux comprenant des cellules souches dans la préparation d'un médicament destiné à favoriser l'épithélialisation et la formation de néo-derme dans un site de plaie, ladite population de cellules concentrée étant à administrer à un patient en ayant besoin, sans besoin de cultiver les cellules avant que les cellules soient administrées. Par rapport à l'invention proposée dans la présente demande de brevet, ce document se rapporte à l'utilisation de cellules régénératives obtenues depuis un tissu adipeux comprenant des cellules souches pour la formation de néo-derme dans un site de la plaie, tandis que l'invention proposée ici se rapporte au fait d'utiliser des cellules souches de la même lignée embryonnaire, d'ectoderme, afin qu'il y ait plus de cellules multipotentes et unipotentes qui n'ont pas besoin de se différencier, la solution de tissu traitée étant ainsi prête pour l'injection en seulement vingt minutes tout en requérant un minimum de manipulation, en ne requérant pas une chirurgie, en ne laissant aucune séquelle et en étant la procédure la plus économique et rapide.

Le document ES2545385T3 se réfère à une unité de traitement de cellules souches dérivées de tissu adipeux autonome, comprenant : un récipient pour l'extraction de tissu qui est configuré pour recevoir un tissu adipeux non transformé qui est retiré d'un patient, ledit récipient pour l'extraction de tissu étant défini par un système fermé ; un premier filtre est positionné à l'intérieur dudit récipient pour l'extraction de tissu, ledit premier filtre ayant une taille de pores d'environ 20 microns à environ 5 mm et ledit premier filtre étant configuré pour retenir un premier composant dudit tissu adipeux non transformé et pour laisser passer un deuxième composant du tissu adipeux non transformé, de sorte que ledit premier filtre sépare ledit premier composant dudit deuxième composant, et ledit premier composant comprenant une population de cellules comprenant des cellules souches dérivées de tissu adipeux et ledit deuxième composant comprenant des lipides adipeux, du sang, des adipocytes matures, et une solution saline ; un récipient pour l'extraction des cellules, qui est configuré pour recevoir ledit premier composant comprenant une population de cellules qui comprend des cellules souches dérivées de tissu adipeux depuis ledit récipient, pour l'extraction de tissu, ledit récipient pour l'extraction de cellules étant placé à l'intérieur dudit système fermé ; un conduit configuré pour permettre le passage dudit premier composant comprenant une population de cellules comprenant des cellules souches dérivées de tissu adipeux depuis ledit récipient, pour l'extraction du tissu dans ledit récipient pour l'extraction des cellules tout en conservant un système fermé ; un concentrateur de cellules positionné à l'intérieur dudit récipient pour l'extraction des cellules, qui est configuré pour faciliter la concentration dudit premier composant comprenant une population de cellules comprenant des cellules souches dérivées de tissu adipeux pour obtenir une population concentrée de cellules comprenant des cellules souches dérivées le tissu adipeux, ledit concentrateur de cellules comprenant une centrifugeuse ou un filtre à membrane qui tourne ; et un orifice de sortie configuré pour permettre le retrait aseptique de ladite population concentrée de cellules comprenant des cellules souches dérivées de tissu adipeux. Dans ce cas, le procédé proposé par la présente invention est bien plus rapide, utilise des cellules souche de même lignée embryonnaire, de l'ectoderme, de sorte qu'il y a des cellules plus unipotentes et multipotentes qui n'ont pas besoin de se différencier, le traitement étant prêt en seulement 20 minutes et la solution tissulaire étant prête pour l'injection, avec des manipulations minimales, sans qu'il soit besoin de chirurgie, ne laissant aucune séquelle et étant beaucoup plus économique.

Conclusions : comme cela ressortit des investigations menées, aucun des documents trouvés ne résout les problèmes posés, au contraire de ce que permet l'invention proposée.

### DESCRIPTION DE L'INVENTION

Le système pour la régénération de muscles, de tendons, de cartilage et d'os à base de micro greffes autologues de cellules souches à partir de la préparation sécurisée et standardisée d'échantillons et de l'automatisation et la désintégration mécanique subséquente de tissus humains solides, comprenant une machine et un kit, la machine étant une unité compacte qui fonctionne conjointement avec le kit et qui fonctionne à une vitesse constante d'environ quatre-vingts tours par minute, tandis que le kit est un dispositif médical à usage unique contenant une grille stationnaire en acier inoxydable ayant environ cent orifices hexagonaux et, autour de chaque orifice, six microlames de coupe des tissus durs et mous lors du passage d'une hélice en acier inoxydable qui est en rotation et qui fait que les tissus se désagrègent au niveau desdits orifices et qui sont filtrés avec une solution ajoutée dans un récipient inférieur, duquel est extrait le contenu cellulaire au moyen d'une absorption avec une seringue.

Le procédé pour la régénération de muscles, de tendons, de cartilage ou d'os à base de microgreffes autologues de cellules souches est basé sur une désagrégation mécanique de tissus autologues contenant des cellules souches et d'autres composants cellulaires du système vasculo stromal (SVE), obtenus par un traitement mécanique des microgreffes autologues au moyen du dispositif breveté sous le titre "dispositif désaggrégateur de matériau biologique pour la préparation de suspensions cellulaires et de microgreffes de tissus", numéro WO 2016/097960 A2.

Pour mettre en oeuvre ce procédé, il est impératif que le patient soit diagnostiqué correctement comme subissant une lésion aigue, non cicatricielle, d'un tendon, d'un muscle, d'un os ou d'un cartilage. Une lésion musculaire et tendineuse ne doit pas dépasser six mois en termes d'évolution, et, une lésion cartilagineuse ne doit pas dépasser le stade de 2-3 de chondromalacie articulaire et le stade de 1-2 d'ostéoarthrose.
a) Dans une première étape, il est procédé au traitement d'un échantillon de tissu provenant d'un donneur, de muscle, de graisse, de derme, de cartilage facial, de préférence extrait de la région postérieure de la conque de l'oreille dans le cas d'un traitement pour la régénération du cartilage, puis à la séparation du derme et de l'épiderme manuellement (desépidermisation) au moyen d'un scalpel ou d'un ciseaux, puis à la découpe de l'autogreffe pour un meilleur traitement mécanique de l'échantillon.
b) Ce tissu est traité dans des conditions maximales de stérilité et est traité dans la machine du système précédemment décrit, en ajoutant au préalable une solution physiologique, en un maximum de 1,2 cc, ou toute autre solution qui agit comme un véhicule pour pouvoir être traitée et injectée par la suite et qui ne détériore pas la viabilité du contenu cellulaire de la suspension et permet ainsi la désagrégation du tissu et le filtrage des cellules souches.
c) Une fois la préparation obtenue, il est possible de procéder à l'utilisation de la solution obtenue au moyen du système précédemment décrit, dans une période de temps non supérieure à une demi-heure si possible, afin de ne pas diminuer la viabilité du contenu cellulaire, au moyen d'une injection intra articulaire en cas de chondromalacie ou d'ostéoarthrose, ou par injection sous-cutanée, le plus près possible de la lésion tendineuse ou musculaire.

La présente invention concerne également l'utilisation de microgreffes autologues de cellules souches et de cellules du système vasculo stromal obtenues par le système et par le procédé décrits ci-dessus pour la régénération de muscles, de tendons, de cartilage et d'os.

### BRÈVE DESCRIPTION DES DESSINS

Pour une meilleure compréhension de la description du procédé, il est annexé un dessin représentant un mode de réalisation préféré de la présente invention. Dans ce dessin schématique, les figures sont les suivantes :

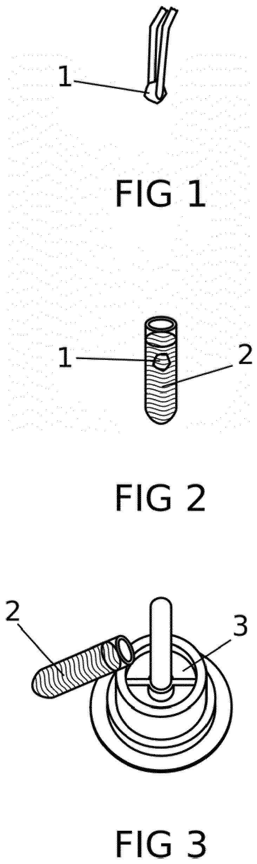

Figure 1 : obtention d'un petit échantillon de tissu (de 1 à 2 mm, jusqu'à 10 mm)
Figure 2 : mouillage avec une solution physiologique stérile
Figure 3 : positionnement du tissu avec 1 ml d'une solution physiologique stérile sur la grille

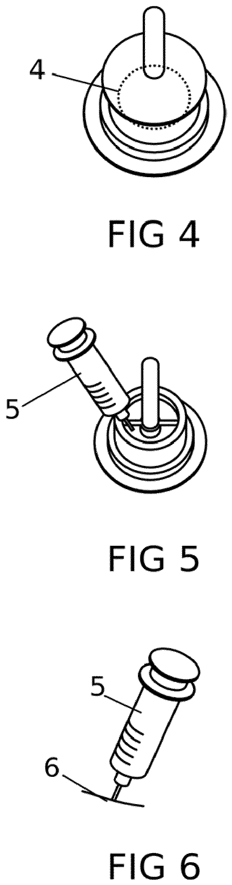

Figure 4 : couverture du filtre et activation de la rotation pour séparer le tissu
Figure 5 : collecte de la suspension de microgreffes avec une seringue dans le trou prévu à cet effet
Figure 6 : application immédiate. Selon la finalité du traitement, elle peut être appliquée seule, avec un PRP (Plasma Riche en Plaquettes) ou un pré-mélange avec un biomatériau.

Les références numériques correspondent aux éléments suivants de l'invention :
- 1.: Échantillon de tissu
- 2.: Solution physiologique stérile
- 3.: Grille
- 4.: Filtre
- 5.: Seringue
- 6.: Application

### DESCRIPTION D'UN MODE DE REALISATION PREFERE

Un mode de réalisation préféré de la présente invention peut être basé sur un système pour la régénération de muscles, de tendons, de cartilage ou d'os à base microgreffes autologues de cellules souches à partir de la préparation sécurisée et standardisée d'échantillons (1) pour l'automatisation et la désintégration mécanique de tissus humains solides. Ce système est constitué par une machine et un kit. La machine est une unité compacte qui fonctionne conjointement avec le kit, fonctionnant à une vitesse constante d'environ quatre-vingts tours par minute, tandis que le kit est un dispositif médical à usage unique comprenant une grille stationnaire (3) en acier inoxydable ayant environ cent orifices hexagonaux et, autour de chaque orifice, six microlames pour la coupe de tissus durs et mous lors du passage d'une hélice en acier inoxydable qui est en rotation et qui fait que le tissu se désagrège au niveau desdits orifices et est filtré en même temps que la solution ajoutée à un récipient inférieur, duquel le contenu cellulaire est extrait par absorption avec une seringue.

Le procédé pour la régénération de muscles, de tendons, de cartilage ou d'os à base de microgreffes autologues de cellules souches est basé sur la désagrégation mécanique de tissus autologues tel que derme, muscle, périchondre et cartilage, de cellules souches et d'autres composants cellulaires du système vasculo stromal (SVE), obtenus par le traitement mécanique des micro greffes autologues au moyen du dispositif breveté sous le titre "dispositif désaggrégateur de matériau biologique pour la préparation de suspensions cellulaires et de microgreffes de tissus", numéro WO 2016/097960 A2.

Pour mettre en oeuvre ce procédé, il est impératif que le patient soit diagnostiqué correctement comme subissant une lésion aiguë tendineuse, musculaire, ou une ostéomalacie de stade 1 et 2 ou une ostéo-arthrose en stade initial.
a) Dans une première étape, il est procédé à un traitement d'un échantillon de tissu provenant d'un donneur, de muscle, de graisse, de derme, de cartilage facial, extrait de préférence de la région postérieure de la conque de l'oreille dans le cas d'un traitement pour la régénération du cartilage, puis à la séparation du derme et de l'épiderme manuellement (desépidermisation) au moyen d'un scalpel ou d'un ciseaux, puis à la découpe de l'autogreffe pour un meilleur traitement mécanique de l'échantillon.
b) Ce tissu est traité dans des conditions maximales de stérilité et est traité dans la machine du système précédemment décrit, en ajoutant au préalable une solution physiologique, en un maximum de 1,2 cc, ou toute autre solution qui agit comme un véhicule pour pouvoir être traitée et injectée par la suite et qui ne détériore pas la viabilité du contenu cellulaire de la suspension et permet ainsi la désagrégation du tissu et le filtrage des cellules souches.
c) Une fois la préparation obtenue, il est possible de procéder à l'utilisation de la solution obtenue au moyen du système précédemment décrit, dans une période de temps non supérieure à une demi-heure si possible, afin de ne pas diminuer la viabilité du contenu cellulaire, au moyen d'une injection intra articulaire en cas de chondromalacie ou d'ostéoarthrose, ou par injection sous-cutanée, le plus près possible de la lésion tendineuse ou une musculaire.

La présente invention concerne également l'utilisation de microgreffes autologues de cellules souches et de cellules du système vasculo stromal obtenues par le système et le procédé décrits ci-dessus pour la demande médicamenteuse, la thérapie régénérative des traumatismes, la réadaptation ou la médecine du sport.

## Revendications

1. Système pour la régénération de muscles, de tendons, de cartilage ou d'os à base de micro greffes autologues de cellules souches à partir de la préparation sécurisée et standardisée d'échantillons (1) pour l'automatisation et la désintégration mécanique de tissus humains solides, comprenant une machine et un kit, la machine étant une unité compacte qui fonctionne conjointement avec le kit et qui fonctionne à une vitesse constante d'environ quatre-vingts tours par minute, tandis que le kit est un dispositif médical à usage unique contenant une grille stationnaire (3) en acier inoxydable ayant environ cent orifices hexagonaux et, autour de chaque orifice, six microlames de coupe des tissus durs et mous lors du passage d'une hélice en acier inoxydable qui est en rotation et qui fait que les tissus se désagrègent au niveau desdits orifices et qui sont filtrés avec une solution ajoutée dans un récipient inférieur, duquel est extrait le contenu cellulaire au moyen d'une absorption avec une seringue.

2. Procédé pour la régénération de muscles, de tendons, de cartilage ou d'os, avec des micro greffes autologues de cellules souches, progénitrices, et de cellules du système vasculo stromal, basé sur une désagrégation mécanique de tissus autologues tel que derme, muscle, périchondre et cartilage contenant des cellules souches et d'autres composants cellulaires du système vasculo stromal (SVE), obtenus par un traitement mécanique des micro greffes autologues au moyen du dispositif breveté sous le titre "dispositif désaggrégateur de matériau biologique pour la préparation de suspensions cellulaires et de micro greffes de tissus", numéro WO 2016/097960 A2, étant impératif que le patient soit diagnostiqué correctement comme subissant une lésion aiguë tendineuse, musculaire ou d'ostéomalacie de stade 1 et 2 ou d'ostéo-arthrose dans des états initiaux, **caractérisé par** la mise en oeuvre des étapes suivantes :
a) Dans une première étape, il est procédé au traitement d'un échantillon de tissu provenant d'un donneur, de muscle, de graisse, de derme, de cartilage facial, de préférence extrait de la région postérieure de la conque de l'oreille dans le cas d'un traitement pour la régénération du cartilage, puis à la séparation du derme et de l'épiderme manuellement (desépidermisation) au moyen d'un scalpel ou d'un ciseaux, puis à la découpe de l'autogreffe pour un meilleur traitement mécanique de l'échantillon ;
b) Ce tissu est traité dans des conditions maximales de stérilité et est traité dans la machine du système précédemment décrit, en ajoutant au préalable une solution physiologique, en un maximum de 1,2 cc, ou toute autre solution qui agit comme un véhicule pour pouvoir être traitée et injectée par la suite et qui ne détériore pas la viabilité du contenu cellulaire de la suspension et permet ainsi la désagrégation du tissu et le filtrage des cellules souches ;
c) Une fois la préparation obtenue, il est possible de procéder à l'utilisation de la solution obtenue au moyen du système selon la revendication 1, dans une période de temps non supérieure à une demi-heure si possible, afin de ne pas diminuer la viabilité du contenu cellulaire, au moyen d'une injection intra articulaire en cas de chondromalacie ou d'ostéo-arthrose, ou par injection sous-cutanée, le plus près possible de la lésion tendineuse ou musculaire.

3. Utilisation de microgreffes de cellules souches autologues et de cellules du système vasculo stromal obtenues par le système et par le procédé selon les revendications 1 et 2, pour la thérapie régénérative traumatologique, la réadaptation ou la médecine du sport.
